# EUROPEAN PATENT APPLICATION

(11) **EP 1 832 302 A1**
(43) Date of publication of application: **12.09.2007**
(21) Application number: 05820158.3
(22) Date of filing: 21.12.2005
(51) Int. Cl.: A61L 31/00, A61L 17/00, A61L 27/00, C08G 69/44, C08L 77/12

(54) **BIODEGRADABLE AND BIORESORBABLE MATERIAL FOR MEDICAL USE**

(30) Priority: 24.12.2004 JP 2004373964
(71) Applicant: Goodman Co., Ltd., Nagoya-shi Aichi 465-0032 (JP)
(72) Inventor: SHIRAHAMA, Hiroyuki, Higashihiroshima-shi, Hiroshima 739-0023 (JP); MIYAZAKI, Masamitsu, Okazaki-shi, Aichi 444-0007 (JP); FUKUCHI, Mikio, Himeji-shi, Hyogo 670-0884 (JP)
(74) Representative: Eisenführ, Speiser & Partner
(86) International application number: PCT/JP2005/023464
(87) International publication number: WO 2006/068168

(57) **Abstract**

Disclosed is a biodegradable and bioresorbable material for medical use which has excellent degradability, heat resistance and mechanical resistance. The biodegradable and bioresorbable material for medical use comprises a polymer blend of a type A polymer and a type B polymer having the following formulae, respectively: type A polymer: type B polymer: wherein each of X₁ and X₂ is 0 to 50 mol%; each of Y₁ and Y₂ is 0 to 50 mol%; each of Z₁ and Z₂ is 50 to 100 mol%; each of m₁ and m₂ is an integer of 3 to 8; R₁ and R₃ independently represent a hydrogen atom or an alkyl group having 1 to 4 carbon atoms; R₁' and R₃' independently represent an alkyl group having 1 to 2 carbon atoms; and R₂, R₂', R₄ and R₄' independently represent an alkyl group having 1 to 4 carbon atoms, provided that both of X₁ and X₂ should not be 0 mol%.

## Description

### (Technical Field)

The present invention relates to a biodegradable bio-absorbable material of a bio-absorbable polymer for clinical practice, which can be used for a medical device made of biodegradable bio-absorbable material, such as suture thread, vascular stent, biological cell carrier, and carriers of drug and the like, and a method for producing the same.

### (Background Art)

Bio-absorbable polymers for use as medical materials such as vascular stent and suture thread include for example polylactic acid, polyglycolic acid, a copolymer of the two, namely polyglactin, polydioxanone, and polyglyconate (the copolymer of trimethylene carbonate and glycolide).

Such bio-absorbable polymers are degraded and absorbed in biological organisms. Therefore, such bio-absorbable polymers are widely used. Because the dynamic properties thereof such as tensile strength and the degradation rate thereof for absorption are individually nearly definite, the bio-absorbable polymers turn fragile when the dynamic properties are enhanced, involving the reduction of the degradation rate. When the degradation rate is increased, alternatively, the dynamic properties are deteriorated. Thus, disadvantageously, the bio-absorbable polymers have only limited purposes for use and are applied to limited sites.
Nonpatent literature 1: KOBUNSHI RONBUNSHU, Vol. 56, No. 9, pp. 550-556 (September, 1999).

### (Disclosure of Invention)

Although PLLA (polylactic acid) is well known as a biodegradable material and mechanical properties of PLLA are superior to other biodegradable materials, PLLA does not have enough heat resistance and enough degradability. Under such circumstances, although P(L-LA (L-lactide) / CL (ε -caprolactone)), P(L-LA / DMO (cyclic depsipeptide)) and P(L-LA / CL / DMO) are reported to be a copolymer having a increased degradability compared to PLLA (see Kobunshi Ronbunshu, Vol.56, No.9, pp.550-556 (September, 1999)), CL contributes to the degradability with significantly reduced heat resistance and mechanical properties, while DMO contributes to the degradability but does not contribute to the heat resistance and the mechanical properties as such. The term "degradability" means a period of time enough for the biodegradable material to be degraded in vivo, preferably short. The term "heat resistance" means that the biodegradable material has a temperature appropriate for forming a medical device commercially and a glass transition temperature appropriate for maintaining various properties at a storage environment and strength at an intravital temperature. The term "mechanical properties" means a biodegradable material having a high strength enough to allow it to function in vivo as medical devices.

The present inventors found that mechanical and thermal properties are unexpectedly improved by mixing two kinds of polymers forming a stereocomplex with each other, and brought the present invention to completion. That is, the present invention provides a biodegradable bio-absorbable material for clinical practice, which comprises the following A-type polymer and B-type polymer: 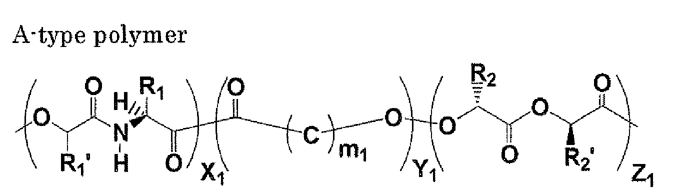 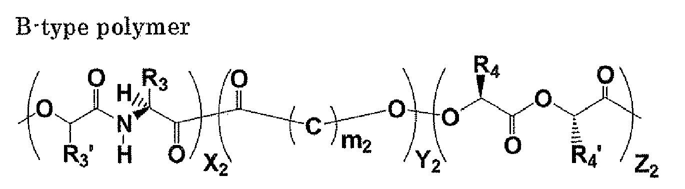 wherein X₁ and X₂ are in the range of 0 to 50 mole%, Y₁ and Y₂ are in the range of 0 to 50 mole%, Z₁ and Z₂ are in the range of 50 to 100 mole%, m₁ and m₂ are in the range of 3 to 8, R₁ and R₃ are the hydrogen or the C1-4 alkyl group, R₁' and R₃' are the C1-2 alkyl group, and R₂, R₂', R₄ and R₄' are the C1-4 alkyl group provided that x₁ and x₂ are not 0 mole% at the same time.

### (Best Mode for Carrying out the Invention)

In the above material (polymer blend) comprising the A-type polymer and B-type polymer, x₁ and x₂ may differ from each other and preferably are in the range of 1 to 20 mole%. y₁ and y₂ may also differ from each other and preferably are in the range of 1 to 30 mole%. z₁ and z₂ may also differ from each other and preferably are in the range of 70 to 99 mole%. m₁ and m₂ may also differ from each other and preferably are in the range of 5 to 7 and most preferably 5.

In the polymer blend comprising the A-type polymer and B-type polymer, R₁, R₃, R₁' and R₃' may differ from each other and preferably are the methyl or ethyl group and more preferably are the methyl group. R₁' and R₃' may or may not have an optical activity. R₂, R₂', R₃ and R₃' may differ from each other and preferably are the methyl or ethyl group and more preferably are the methyl group.

In the polymer blend comprising the A-type polymer and B-type polymer, the A-type polymer and B-type polymer may have molecular weights different from each other and their number average molecular weights are preferably in the range of 1 ×10³ to 3 ×10⁵, more preferably in the range of 2 ×10³ to 3 ×10⁵ and even more preferably 2 ×10⁴ to 2 ×10⁵, in light of the formation of a stereocomplex.

In the polymer blend comprising the A-type polymer and B-type polymer, the A-type polymer and B-type polymer may be either a random copolymer or a block copolymer and preferably the random copolymer in light of the thermal and mechanical properties.

In the polymer blend comprising the A-type polymer and B-type polymer, the blend ratio of A-type polymer and B-type polymer is preferably 1:9 to 9:1 and more preferably 3:7 to 7:3.

A method for synthesis of the A-type polymer and B-type polymer includes as follows. A predetermined amount of the following monomers purified in a suitable way, 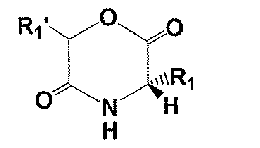 wherein R₁ is the hydrogen or C1-4 alkyl group, R₁' is the C1-2 alkyl group; 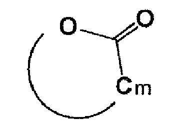 wherein m is in the range of 3 to 8; and 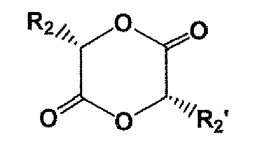 or 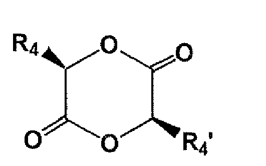 wherein R₂, R₂', R₄ and R₄' are the C1-4 alkyl group, are added to a reactor, and furthermore a catalyst is added to the reactor in the amount needed for the monomers (generally 10⁻⁷ to 10⁻³ mol per mol of the monomers). When a copolymer is prepared, two kinds of the above monomers are used, and when a tercopolymer is prepared, three kinds of the above monomers are used. A polymerization catalyst generally used includes a metallic catalyst such as tin octylate. Furthermore, a higher alcohol is added to the reactor as an initiator. The monomers, catalyst and initiator are adequately stirred in the reactor and polymerized at the temperature of 120 to 200°C under inert gases. A polymerizing temperature, the amount of catalyst and polymerizing time are controlled depending on a desired molecular weight. After the completion of polymerization, the resulting polymers are dissolved in an organic solvent such as chloroform and purified by reprecipitation using methanol or the like.

The catalyst at the end of the polymer can also be removed by adding the number of moles of hydrochloric acid not less than that of the catalyst used in the polymerization to form a metallic salt during purification.

A method for blending the polymers includes a method in which the L-DMO/L-LA copolymer and the L-DMO/D-LA copolymer or the L-DMO/CL/L-LA tercopolymer and the L-DMO/CL/D-LA tercopolymer, which are reprecipitated by using methanol, are dissolved in an adequate amount of chloroform at the predetermined ratio of the polymers again and then they are mixed, briskly stirred for 1 to 3 hours, transferred into a teflon petri dish, and cast to obtain the polymer blend (stereocomplex) in the form of a film. Alternatively, the polymers may be dissolved in chloroform after the polymerization and reprecipitated by using methanol to obtain the polymer blend (stereocomplex). In this connection, the above reprecipitation removes lower molecular weight polymers which do not form the stereocomplex.

### [Examples]

So as to describe the invention in more detail, the invention is now described with reference to the attached drawings.

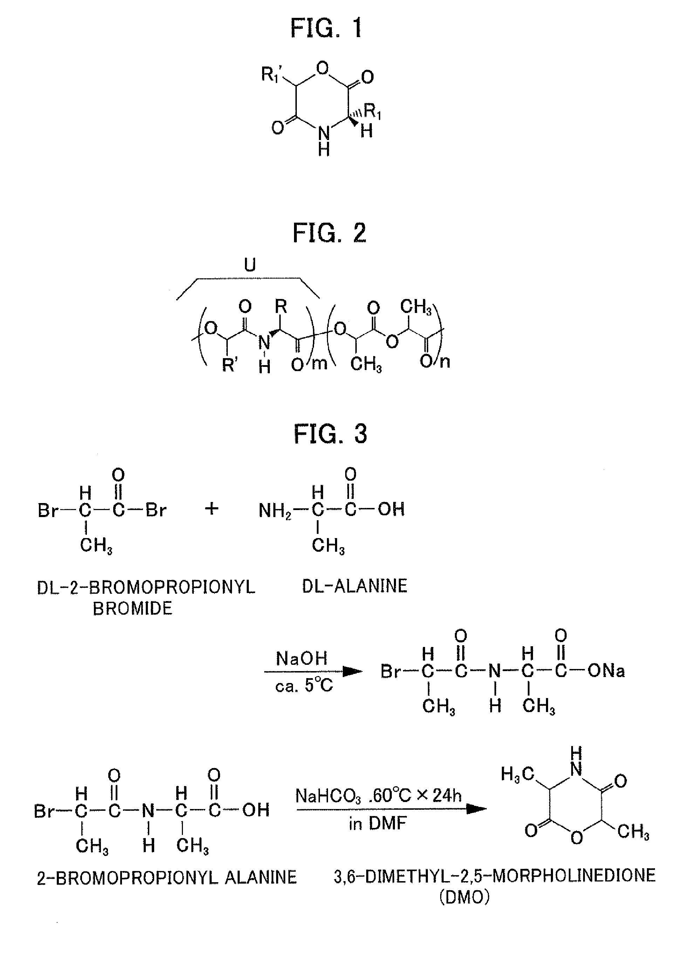

The structure of the depsipeptide is shown in Fig. 1.

As shown in the figure, the R₁ group in a side chain is a C1-4 alkyl group, while the R₁' group in a side chain is a C1-2 alkyl group.

Concerning examples of the depsipeptide, depsipeptides are synthesized from amino acid and a hydroxylate derivative, using chloroacetyl chloride, 2-bromopropionyl bromide and 2-bromo-n-butyryl bromide are as the hydroxylate derivative to prepare depsipeptides, namely L-MMO, L-DMO, and L-MEMO, in the order of the hydroxylate derivatives. All of them are applicable to the invention. The enzymatic degradation level of a copolymer from such depsipeptide monomer and a bio-absorbable polymer ε-caprolactone (CL) with proteinase K is in the order of L-MMO/CL > L-DMO/CL > L-MEMO/CL.

As to the depsipeptide synthesized from amino acid and an oxyacid derivative, amino acids such as L-alanine, L-(DL- or D-)valine, and L-leucine are used to prepare depsipeptides, namely DMO, PMO and BMO in the order of the amino acids. All of them are applicable to the invention. The enzymatic degradation level of a copolymer from such depsipeptide monomer and a bio-absorbable polymer ε-caprolactone (CL) with proteinase K is in the order of DMO/CL > PMO/CL ≥ BMO/CL. The enzymatic degradation level thereof with cholesterol esterase is in the order of PMO/CL > BMO/CL ≥ DMO/CL.

### [synthesis of L-DMO/CL/L-LA random tercopolymer, L-DMO/CL/D-LA random tercopolymer, L-DMO/L-LA random copolymer and L-DMO/D-LA random copolymer]

A tercopolymer was prepared by adding a cyclic depsipeptide (DMO) to a copolymer of L-lactide (L-LA) as a raw material of polylactic acid and ε-caprolactone as a raw material of poly ε-caprolactone.

Fig. 2 is the structure view of the copolymer with the peptide unit as recovered by the polymerization of the depsipeptide. U expresses depsipeptide unit.

Therefore, 3,6-dimethyl-2,5-morpholine-dione (DMO) was synthetically prepared as a cyclic depsipeptide. The cyclic depsipeptide is a cyclic ester amide prepared from α-amino acid and a α-hydroxylate derivative. Herein, DL-alanine and DL-2-bromopropionyl bromide were used as α-amino acid and α-hydroxylate derivative, respectively. Regardless of whether D- or L-alanine was used as α-amino acid, the cyclic depsipeptide was synthesized in a similar way.

At the first step of the synthesis, the Schotten·Baumann reaction between alanine and 2-bromopropionyl bromide was carried out in an aqueous alkaline solution, for peptide linking to prepare 2-bromopropionyl alanine (Fig. 3).

In other words, 150 ml of an aqueous solution of DL-alanine (53.4 g; 0.6 mol) in 4N NaOH (0.6 mol) was cooled to about 5 °C, to which were then added alternately 180 ml of 4N NaOH (0.72 mol) and 69.9 ml of DL-2-bromopropionyl bromide (0.66 mol) under cooling and agitation in an ice bath over about 30 minutes. The reaction mixture was continuously kept at mild alkalinity. After the termination of the reaction, the product in white was filtered and isolated.

The product was dissolved in water, followed by dropwise addition of 5N HCl to about pH 3. Thereafter, water was removed by evaporation. While the remaining aqueous solution was gradually acidified with 5N HCl under cooling, an additional product in white was recovered. These white products recovered were extracted in diethyl ether with a Soxhlet extractor, for purification.

Yield 30 to 40 %; ¹H NMR(δ, CDCl₃) 1.54(d, 3H, NHCHCH₃), 1.91(d, 3H, BrCHCH₃), 4.45(q, 1H, NHCHCH₃), 4.59(q, 1H, BrCHCH₃), 6.88(brs, 1H, NH). Continuously, the purified 2-bromopropionyl alanine (19.7 g; 0.0881 mol) and the equimolar NaHCO₃ (7.40 g; 0.0881 mol) were added to 150 ml of dimethylformamide (DMF).

Then, the resulting mixture was refluxed at 60 °C for 24 hours, for intramolecular cyclization desalting, to recover a cyclic depsipeptide DMO in white powder (Fig. 3).

DMO was purified via recrystallization twice in chloroform.

Yield 40 to 60 %; mp 158 to 159°C; 1H NMR(δ, CDCl₃) 1.54(d, 3H, NHCHCH₃), 1.62(d, 3H, OCHCH₃), 4.24(q, 1H, NHCH), 4.91(q, 1H, OCH), 7.07ppm (brs, 1H, NH).

The synthesis of the tercopolymer is now described.

Among the copolymerizable monomers, the cyclic depsipeptide (L-DMO) was synthetically prepared from α-amino acid (L-alanine) and a α-hydroxylate derivative (DL-2-bromopropionyl bromide), and was then purified for use.

Further, lactone (CL) was purified by dissolving CL in toluene and subsequently drying CL with CaH₂ for 48 hours, and then subjecting the resulting CL to distillation under reduced pressure (twice). L-Lactide (L-LA) was purified by recrystallization in THF and sublimation (twice).

All the polymerization procedures were done in argon atmosphere.

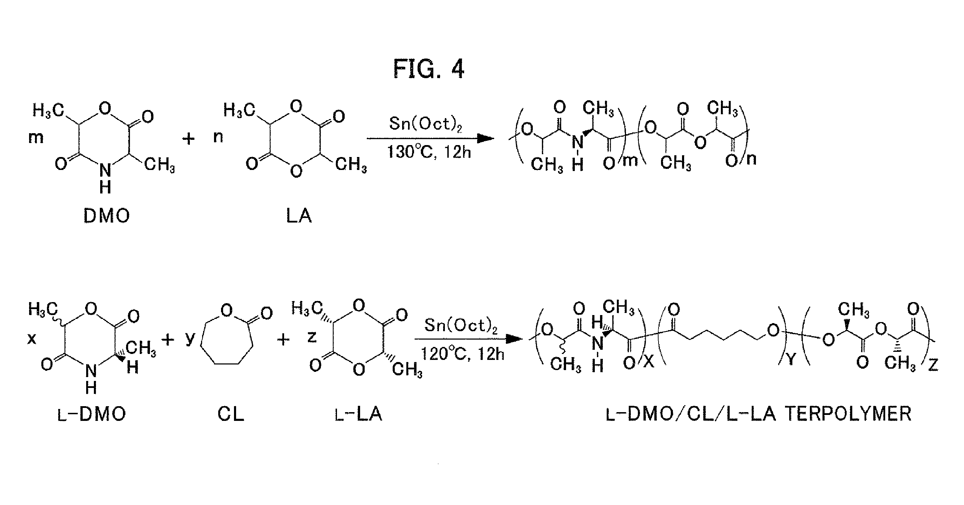

The synthetic scheme of the L-DMO/CL/L-LA tercopolymer is shown in Fig. 4.

The copolymer was prepared as follows.

Given amounts of both the monomers L-DMO and L-LA dissolved in THF and a toluene solution of a catalytic amount of tin (II) octylate [Sn(Oct)₂; 0.2 mol %/monomer] are charged in a Schrenk tube (polymerization container), from which the solvents THF and toluene are subsequently trapped and removed under reduced pressure.

Then, a given amount of the CL monomer is placed in the same polymerization container, which is then sealed. The sealed container was immersed in an oil bath at 120 °C, to initiate the polymerization.

After a given period of time (12 hours), the polymerization container was taken out of the oil bath and then cooled. The resulting crude polymer was dissolved in chloroform, an amount of hydrochloric acid more then double that of charged tin octylate was added to the polymer and stirred (for more than 3 minutes) to remove the catalyst at the end of the polymer, and the generated metal salt was extracted in distilled water (more than one) and purified via reprecipitation in methanol after dehydration. Tables 1 and 2 show the yields and molecular weights of the resulting polymers.

Further, the ¹H-NMR data (δ, CDCl₃) of the tercopolymer L-DMO/CL/L-LA (= 8:13:79) is as follows.
1.38(m, 2H, CH₂CH₂CH₂CH₂CH₂), 1.50(m, 6H, CH₃×2(L-DMO)), 1.57(d, 6H, CH₃×2(L-LA)), 1.68(m, 4H, CH₂CH₂CH₂CH₂CH₂), 2.25 to 2.45(splitting in two peaks, 2H, CCH₂), 4.60(m, 1H, OCH(L-DMO)), 5.17(q, 3H, OCH× 2(L-LA), NHCH(L-DMO)), 6.60ppm(br.m, 1H, NH).

Using two or three kinds of monomers to be polymerized, L-DMO/CL/D-LA random tercopolymer, L-DMO/L-LA random copolymer and L-DMO/D-LA random copolymer were synthesized by a method similar to the one above. The ¹H-NMR datas (δ, CDCl₃) of the resulting polymers are as follows.
P(L-DMO/L-LA): 1.48(m, 6H, CH₃×2(L-DMO)), 1.57(d, 6H, CH₃×2(L-LA)), 4.59(m, 1H, NHCH), 5.17(m, 1H, OCH), 6.56ppm(br. d, 1H, NH). P(L-DMO/D-LA): 1.49(m, 6H, CH₃×2(L-DMO)), 1.57(d, 6H, CH₃×2(L-LA)), 4.61(m, 1H, NHCH), 5.17(m, 1H, OCH), 6.58ppm(br. d, 1H, NH). P(L-DMO/CL/D-LA): 1.38(m, 2H, CH₂CH₂CH₂CH₂CH₂), 1.50(m, 6H, CH₃×2(L-DMO)), 1.57(d, 6H, CH₃×2(L-LA)), 1.68(m, 4H, CH₂CH₂CH₂CH₂CH₂), 2.26~2.38(splitting in two peaks, 2H, CCH₂), 4.59(m, 1H, OCH(L-DMO)), 6.66ppm(br. m, 1H, NH).

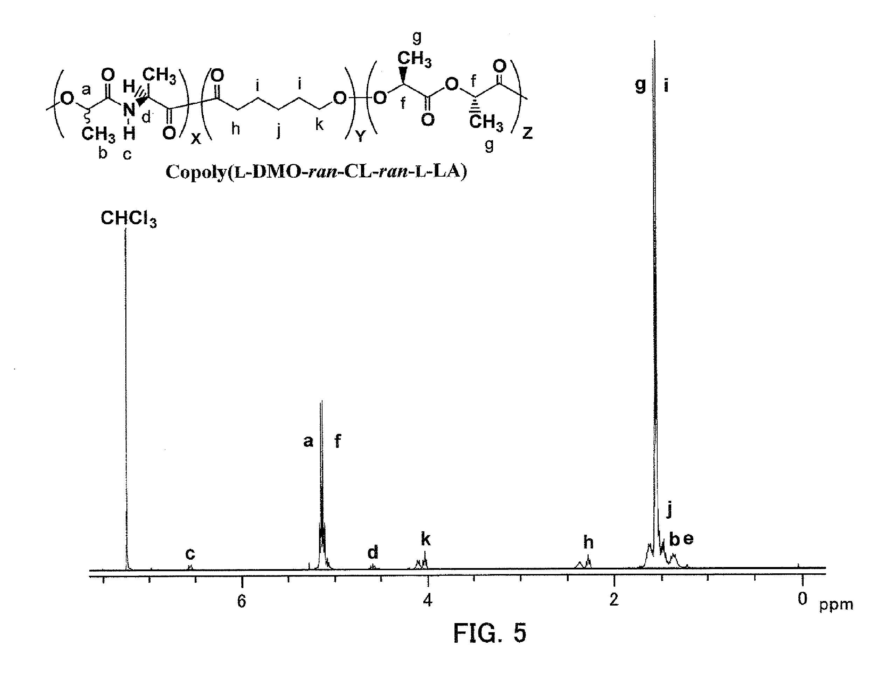

The NMR spectra of the above copolymers and tercopolymer are shown in Figs. 5 to 8.

The composition of the copolymer was determined on the basis of the peak integration ratio of ¹H NMR spectrum measured with a 400 MHz magnetic resonance system (JEOL JMN-LA400). Based on the spectrum, the chain sequence (randomness) of the copolymer was deduced as well.

The number average molecular weight (Mn) of the polymer and the molecular distribution (Mw/Mn) thereof were determined on the basis of a standard curve prepared from standard polystyrene, using GPC 8010 system manufactured by TOSO, Co., Ltd. [column: TSK Gel (G2000H_{HR}+G3000H_{HR}+ G4000H_{HR}+G5000H_{HR}), column temperature of 40°C and differential refractive index (RI) meter]. Chloroform was used as the eluent at the flow of 1 mLmin⁻¹.

### [method for preparing the polymer blend]

After the same amount of two kinds of polymers (L-DMO/CL/D-LA and L-DMO/CL/L-LA, or L-DMO/L-LA and L-DMO/D-LA) purified via reprecipitation in methanol, were dissolved in an appropriate amount of chloroform, these were mixed and briskly stirred for 1 to 3 hours, and then transferred into a teflon petri dish and cast to obtain the polymer blend in the form of a film.

### [Thermal and mechanical properties and enzymatic degradation profiles of the polymer blend]

The thermal properties of the polymer and the polymer blend, namely the glass transition temperature (Tg), melting point (Tm) and melting heat (Δ Hm) thereof were measured, using a differential scanning calorimeter SSC5100 DSC22C manufactured by Seiko Electric Co., Ltd. The measurement was done in nitrogen atmosphere at a temperature elevation rate of 10 °C/min.

The mechanical properties of the polymer and the polymer blend (tensile strength and elongation at breaking) were measured, using a tensile tester AGS-H 100N manufactured by Shimadzu Corporation at a crosshead speed of 100 mm/min and a gage length of 15 mm. The measurement was done, in average, at least at three times. Additionally, a dumbbell test piece (parallel length × width × thickness = 40 × 40 × 0.2 mm) of a polymer sample was prepared by pressing the polymer material under heating at 180 to 200 °C for about 5 minutes (type SDMP-1000-D, gage JISK-7162-5B).

The enzymatic degradation test of the polymer and the polymer blend was done in the same manner as in the related art. The test is now summarized below.

Polymer film (film thickness of about 200 µm; several tens grams) sealed in polyethylene sheet mesh (mesh size of about 1 × 1 mm) was incubated (37 °C) in a sample tube bottle placing an enzyme and a buffer (50 ml) therein, for degradation. The enzyme concentration was 1 International Unit (IU) per 1 mg of the polymer sample.

Herein, the buffer containing the enzyme (degradation solution) was exchanged to a fresh one every about 40 hours, taking account of the reduction of the oxygen activity and the contamination and growth of microorganisms in air.

The degradation level was evaluated on the basis of the changes of the weight and physico-chemical properties (molecular weight, composition and thermal properties) of the polymer before and after degradation. Proteinase K (derived from Tritirachium album; manufactured by Merck Ltd., Japan; oxygen activity of 30.0 mAnsonU/mg) was used as one of proteases, while Tricine (pH 8.0) was used as Good's buffer.

The thermal and mechanical properties of each polymer and polymer blend are shown in Tables 1 and 2. The changes of the thermal and mechanical properties depending on the blending ratio are shown in Figs. 9 and 10, and the results of enzymatic degradation profiles are shown in Fig 11.

### [Table 1]

**Table 1**

| Polymer | P(L-DMO/LA) | | Yield | Mn | Mw/Mn | Tg | Tm 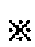 ¹ | ΔHm 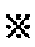 ² | Strength | Modulus | Elongation |
|---|---|---|---|---|---|---|---|---|---|---|---|
| | (feed mole ratio) | (measured mole ratio) | (%) | 10⁴ | | (°C) | (°C) | (Jg^{.1}) | (MPa) | (MPa) | (%) |
| P(L-DMO/L-LA) | 5/95 | 3.8/96.2 | 76.3 | 4.2 | 1.8 | 60.5 | 168 | 42.4 | 50.2 | 620.5 | 10.6 |
| P(L-DMO/D-LA) | 5/95 | 3.4/96.6 | 71.0 | 2.4 | 1.8 | 47.3 | 157 | 23.1 | 49.5 | 604.7 | 9.1 |
| L+D-LA/DMO Stereocomplex | 40/60 blend | | | | | 24.0 | (150),215 | (0.5), 18.2 | 59.1 | 830.8 | 11.2 |
| | 50/50 blend | | | | | 25.0 | (154), 212 | (0.6), 37.5 | 61.5 | 873 | 11.2 |
| | 70/30 blend | | | | | 29.2 | (166), 213 | (3.3), 27.8 | 62.1 | 499.9 | 14.2 |

### [Table 2]

**Table 2**

| Polymer | P(L-DMO/CL/LA) | | Yield | Mn | Mw/Mn | Tg | Tm 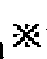 ¹ | ΔHm 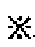 ² | Strength | Modulus | Elongation |
|---|---|---|---|---|---|---|---|---|---|---|---|
| | (feed mole ratio) | (measured mole ratio) | (%) | 10^{4.} | | (°C) | (°C) | (Jg^{.1}) | (MPa) | (MPa) | (%) |
| P(L-DMO/CL/L-LA) | 5/25/70 | 4.4/21.8/73.8 | 75.0 | 4.7 | 1.7 | 19.9 | 132 | 16 | 17.6 | 66.6 | 404.4 |
| P(L-DMO/CL/D-LA) | 5/25/70 | 5.1/19.6176.3 | 60.0 | 2.8 | 1.7 | 3.9.7 | 113 | 9.4 | 5.6 | 25.3 | 537.1 |
| L+D-LA/CL/DMO Stereocomplex | 40/60 blend | | | | | -2.0 | (117), 178 | (1.8), 8.8 | 20.6 | 80.9 | 389.1 |
| | 50/50 blend | | | | | 4.8 | (125), 177 | (5.7), 7.6 | 31.5 | 113.9 | 390.8 |
| | 70/30 blend | | | | | 2.3 | (120), 171 | (2.1), 12.4 | 26.5 | 102.4 | 285 |

| | | | | | | | | | | | |
|---|---|---|---|---|---|---|---|---|---|---|---|
| 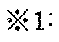 Two values may be shown about melting point (Tm) or melting heat (Δ Hm). This is because the melting points (Tm) or melting heats (Δ Hm) of the copolymer (value in parentheses) and stereocomplex were measured and observed. 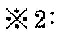 Having compared melting heat (Δ Hm) of the copolymer (value in parentheses) to that of the stereocomplex, it was found that of the stereocomplex is larger than that of the copolymer and it was found that the majority of the polymer blend is the stereocomplex. | | | | | | | | | | | |

In the cases of the copolymer and tercopolymer, a single Tg and a single Tm were only observed, as shown in Tables 1 and 2, suggesting intense randomness.

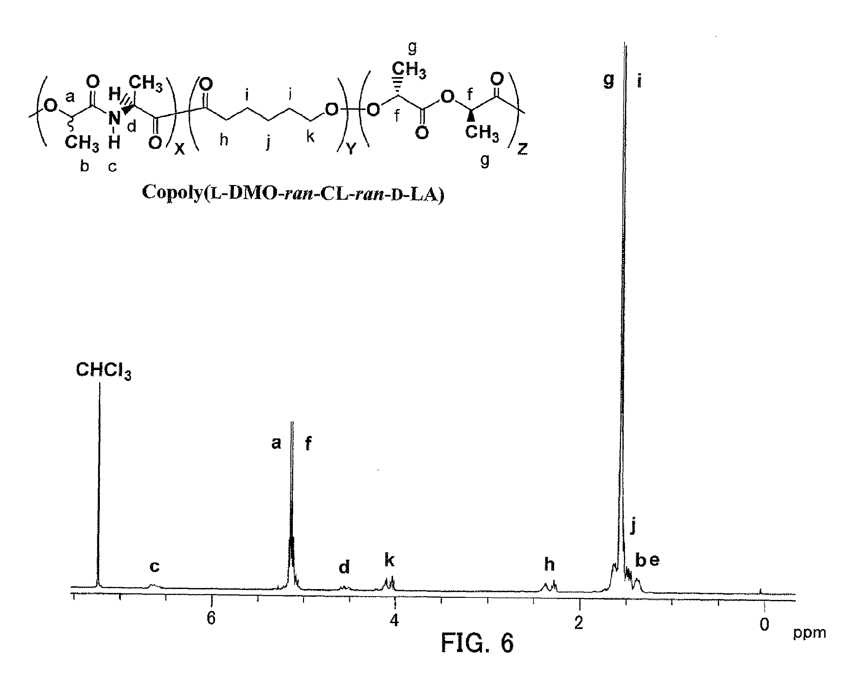

Figs. 5 and 6 depict the ¹H NMR spectrum of the tercopolymer. The chart establishes the verification that the tercopolymer is random. In other words, the proton peaks (k, h) of the α- and ε-methylene in the CL unit are sensitive to the adjacent comonomer units. It is indicated that because these peaks are individually split into two (the peak on the side of high magnetic field corresponds to the homosequence of CL-CL; the peak on the side of low high magnetic field correspond to a peak based on the hetero-sequence of L-LA-CL and L-DMO-CL), the tercopolymer is a random copolymer.

Additionally, the reason why the unit L-DMO is introduced appropriately in the resulting copolymer via copolymerization at 120 °C lower than the Tm thereof (about 170°C) is that the polymerization of the highly reactive L-LA (with Tm of about 95 °C) first occurs and the active elongating terminus induces the ring-opening of the L-DMO (and/or CL), which is then incorporated randomly in the copolymer.

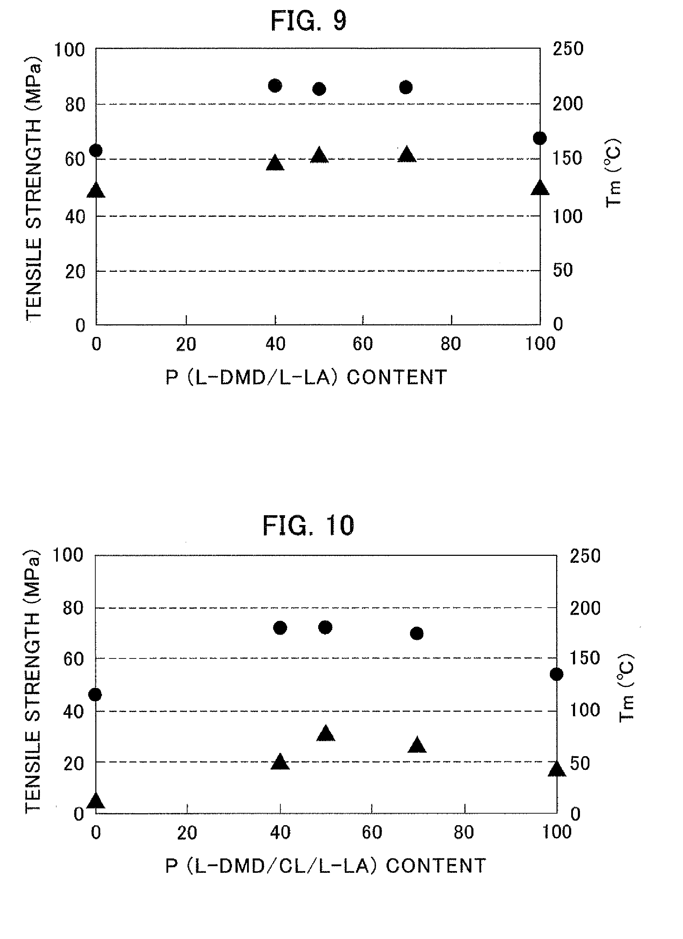

The results shown in Tables 1 and 2 and Figs. 9 to 11 Fig. i2 show that the mechanical and thermal properties of the polymer blend of copolymer and tercopolymer are respectively superior to those of PLLA, the copolymer itself and the tercopolymer itself, and the enzymatic degradation profiles of the polymer blends of copolymer and tercopolymer are respectively superior to those of PLLA.

### (Industrial Applicability)

Polylactic acid, which is a homopolymer or stereocomplex, has a small elongation of mechanical property and thus is restricted due to its breaking or cracking from being used in medical devices which change their form inside the body; for example they expand. However, the bio-absorbable polymer of the invention has a necessary elongation property similar to those of copolymer and tercopolymer without having to sacrifice the strength, thermal property or degradation profiles and thus is appropriate for use in medical devices.

The bio-absorbable polymer of the invention is also able to control the degradation profiles by adjusting the blending ratio of said polymer.

In addition, the bio-absorbable polymer of the invention has a higher biocompatibility for use in indwelling medical devices inside the body and bio-absorbable medical devices compared with polylactic acid (lactide) and caprolactone having no hydrophilic group and the copolymer thereof, because the N-H group of the depsipeptide unit of the bio-absorbable polymer is hydrophile.

### (Brief Description of Drawings)

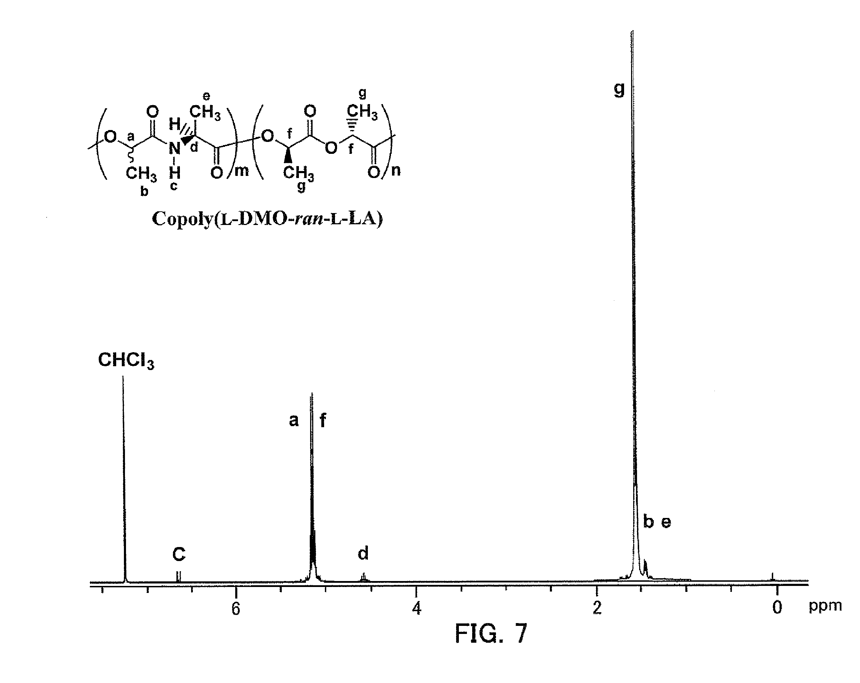

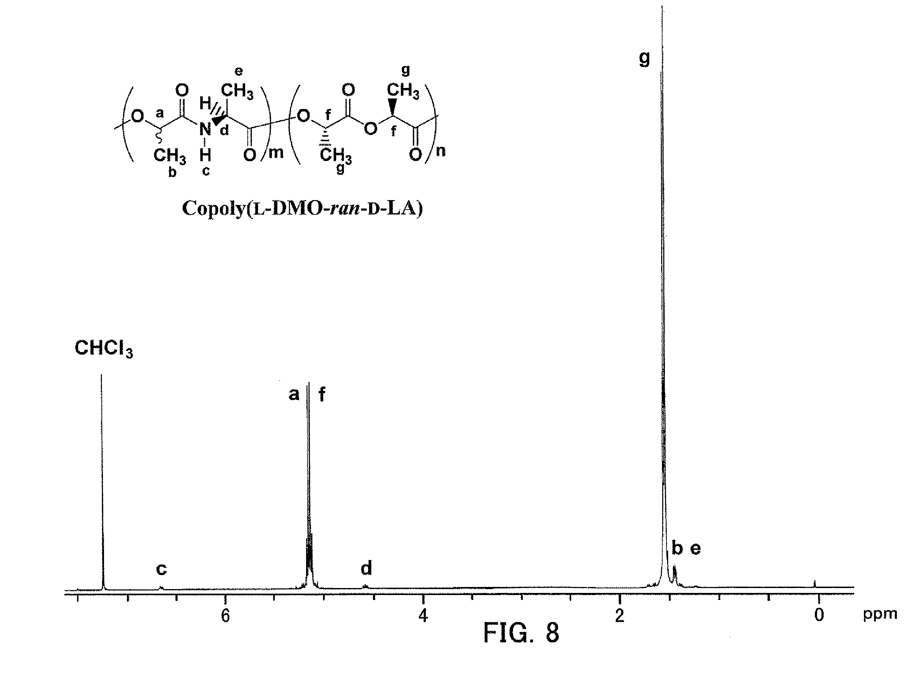

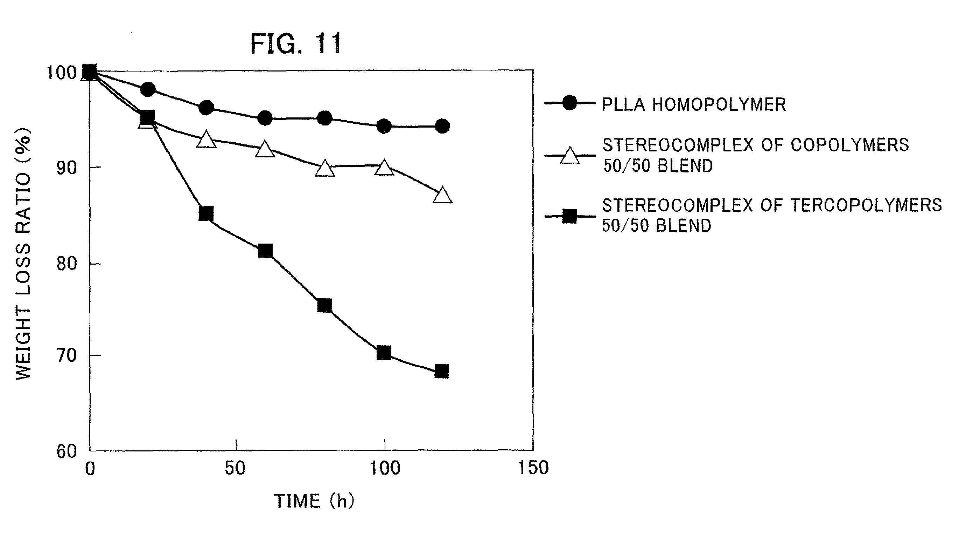

Fig. 1 depicts the structure view of depsipeptide; Fig. 2 depicts the structure view of a copolymer with a depsipeptide unit; Fig.3 depicts the explanatory scheme of the synthesis of the depsipeptide; Fig. 4 depicts the structure views of the copolymer and tercopolymer of a ring-opened and copolymerized depsipeptide; Fig. 5 depicts a chart of a ¹H-NMR spectrum of the L-DMO/CL/L-LA tercopolymer; Fig. 6 depicts a chart of the ¹H-NMR spectrum of the L-DMO/CL/D-LA tercopolymer; Fig. 7 depicts a chart of the ¹H-NMR spectrum of the L-DMO/L-LA copolymer; Fig. 8 depicts a chart of the ¹H-NMR spectrum of the L-DMO/D-LA copolymer; Fig. 9 is a figure depicting changes of the tensile strength and thermal resistance depending on a blend ratio of the copolymers; Fig. 10 is a figure depicting changes of the tensile strength and thermal resistance depending on a blend ratio of the tercopolymers; Fig. 11 depicts a graph of the results of the enzymatic degradation profiles.

## Claims

1. A biodegradable bio-absorbable material for clinical practice, which comprises the following A-type polymer and B-type polymer: 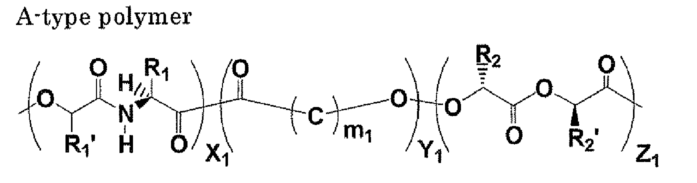 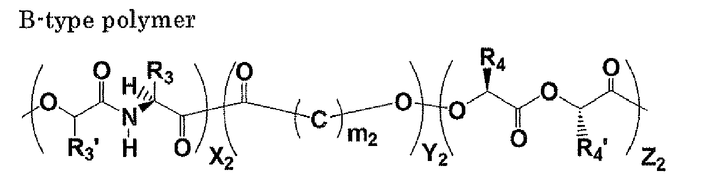 wherein X₁ and X₂ are in the range of 0 to 50 mole%, Y₁ and Y₂ are in the range of 0 to 50 mole%, Z₁ and Z₂ are in the range of 50 to 100 mole%, m₁ and m₂ are in the range of 3 to 8, R₁ and R₃ are the hydrogen or C1-4 alkyl group, R₁' and R₃' are the C1-2 alkyl group, and R₂, R₂', R₄ and R₄' are the C1-4 alkyl group provided that x₁ and x₂ are not 0 mole% at the same time.

2. The biodegradable bio-absorbable material for clinical practice of claim 1 wherein number average molecular weights of A-type polymer and B-type polymer are in the range of 1× 10³ to 3 × 10⁵.

3. The biodegradable bio-absorbable material for clinical practice of claim 1 or 2 wherein A-type polymer and B-type polymer are random copolymer.

4. The biodegradable bio-absorbable material for clinical practice of any of claims 1 to 3 wherein blend ratio of A-type polymer and B-type polymer is in the range of 1:9 to 9:1.
